# EUROPEAN PATENT SPECIFICATION

(11) **EP 3 806 926 B1**
(45) Date of publication and mention of the grant of the patent: **09.09.2026**
(21) Application number: 19820160.0
(22) Date of filing: 13.06.2019
(51) Int. Cl.: A61M 1/36, A61M 60/117, A61B 17/34

(54) **DUAL LUMEN CANNULA WITH FLEXIBLE DISTAL END**
DOPPELLUMENKANÜLE MIT FLEXIBLEM DISTALEN ENDE
CANULE À DOUBLE LUMIÈRE AYANT UNE EXTRÉMITÉ DISTALE FLEXIBLE

(30) Priority: 14.06.2018 US 201862684858 P
(43) Date of publication of application: 21.04.2021
(73) Proprietor: CardiacAssist, Inc., Pittsburgh, PA 15238 (US)
(72) Inventor: KELLY, Patrick, A., North Huntingdon, Pennsylvania 15642 (US); SVITEK, Robert, G., Freeport, Pennsylvania 16224 (US); STOKES, Jerry, Sarver, Pennsylvania 16055 (US)
(74) Representative: Buzzi, Notaro & Antonielli d'Oulx S.p.A.
(86) International application number: PCT/US2019/036987
(87) International publication number: WO 2019/241522

(56) References cited:
- WO-A2-2017/190155
- US-A- 4 808 163
- US-A- 5 827 225
- US-A- 5 976 103
- US-A1- 2005 288 632
- US-A1- 2012 143 123
- US-A1- 2013 158 338
- US-A1- 2013 274 711
- US-B1- 6 287 319
- US-B2- 9 168 352

## Description

### CROSS REFERENCE TO RELATED APPLICATION

The present application claims priority to U.S. Provisional Application No. 62/684,858 filed on June 14, 2018.

### BACKGROUND

### Field

The present disclosure generally relates to devices and methods for assisting a patient's heart with a cannula. More specifically, the present disclosure is related to a cannula assembly having a flexible tip to provide safe and efficient positioning in the patient's vasculature, as well as to methods for assisting a patient's heart with a cannula assembly.

### Description of the Related Art

Examples of existing cannula devices are described in U.S. Patent Nos. 9,168,352, 9,782,534, and 10,279,101. Additional background document include, i.a. US 5,976,103 and US 9,168,352.

Traditional cannulae used for patient life support generally involve single lumen cannulae at multiple insertion sites, high volume circuits, and cannulae that are not capable of long-term use. Multiple insertion sites increase the risk of bleeding, vessel damage, and infection, as well as pain and discomfort to the patient. These cannulae are designed and built for short-term acute therapies. Additionally, traditional cannulae usually require access sites located in the patient's groin area near the right or left femoral veins.

Patients with severe right-sided circulatory and/or right-sided ventricular failure have significantly high mortality and morbidity caused by a multitude of factors in multiple patient populations. Historically, Right Ventricular Assist Devices (RVADs) and Left Ventricular Assist Devices (LVADs) have been adapted for use on surgical patients without a percutaneous or catheter lab option available. These surgical RVADs have been applied on patients with right inferior myocardial infarction, acute right-sided ischemic myocardial infarctions (with large left and right propagation), cardiogenic shock, LVAD-created right ventricular dysfunction, post-transplant right ventricular failure, and pulmonary hypertension. Acute myocardial infarction and cardiogenic shock have been treated with intra-aortic balloon pumps and maximal inotropic support, to which many patients become refractory. Surgically implanted LVADs can create a significant septal shift that leads to a dynamic change in the Starling curve that abruptly places patients into severe right ventricular failure. Patients can limit post-transplant survival bridged to transplant to/from an RVAD with severe right ventricular failure. Secondary pulmonary hypertension leads to an exacerbation of right ventricular failure in acute and chronic situations, which may be treated with RVADs.

Some conventional devices do not have the capability to reach the pulmonary artery (PA) from the internal jugular vein via a percutaneous insertion. Some traditional cannulae are inserted into the patient's heart through a direct access point in the patient's right or left femoral vein. Alternatively, traditional RVADs have a cannula either primarily placed in the PA or a graft sewn onto the PA, then a cannula inserted through the graft. The assembly can then be visualized in the PA via fluoroscopy and X-ray with the aid of distal markers in the cannula, verifying the proper orientation of the outflow to the patient. In these embodiments, the patient's torso length can limit the ability to access the PA via percutaneous insertion. If a cannula is not of a proper length, the interventional procedure may not unload the right ventricle, which leads to an increase of morbidity and mortality.

Furthermore, traditional venoarterial extracorporeal membrane oxygenation (VA ECMO) is the current standard of care used to treat right ventricular failure and respiratory failure percutaneously. A VA ECMO procedure draws blood from the right atrium and pumps it through an oxygenator and back into the arterial circulation via the femoral artery. VA ECMO bypasses the lungs and the heart completely. Therefore, residual blood is left stagnant in both the heart and lungs, potentially leading to thrombosis and an inadequately unloaded right ventricle. Additionally, the arterial cannulation can lead to problems including but not limited to bleeding, stroke, and infection.

Still further, some conventional devices are known to cause abrasion and other injury to the vessel walls, as well as discomfort to the patient due to the rigidity necessary to maneuver through the complex pathways of a patient's vasculature.

### SUMMARY

In view of the foregoing, there is a need for a dual lumen cannula with a single insertion point. There is an additional need for a dual lumen cannula that eliminates multiple access sites and reduces bleeding, vessel damage, and infection, as well as pain and discomfort to the patient. Furthermore, there exists a need for a dual lumen cannula that enables patients to be ambulatory with access sites provided in the neck area instead of the groin.

The invention is defined by the features of independent claim 1.

Embodiments of the present invention are directed to a dual lumen coaxial cannula assembly. The cannula assembly includes a first infusion tube having a first elongate body defining a first lumen therethrough, the first infusion tube having a proximal end, a distal end, and a sidewall extending circumferentially therebetween; a second drainage tube co-axially aligned with the first infusion tube and having a second elongate body with a second lumen defined by a space between the first infusion tube and the second drainage tube, the second drainage tube having a proximal end, a distal end, and a sidewall extending circumferentially therebetween; a connector attached to the proximal end of the first infusion tube and the proximal end of the second drainage tube; and a distal tip defining a distal lumen therethrough, the distal tip having a proximal end, a distal end, and a sidewall extending circumferentially therebetween, the distal tip connected to the distal end of the first elongate body. The distal tip is manufactured of a material having a lower hardness than a hardness of the first elongate body.

In some examples, the distal tip is connected to a distal end of the first elongate body along a plane perpendicular to a longitudinal axis of the first infusion tube.

In some examples, the distal tip and the distal end of the first elongate body are tapered to increase a surface area of a connection between the distal tip and the distal end of the first elongate body.

In some examples, the distal tip and the distal end of the first elongate body are roughened to increase a surface area of a connection between the distal tip and the distal end of the first elongate body.

According to the claimed invention, the sidewall of the first infusion tube defines a shoulder such that a portion of the sidewall of the first infusion tube extending distally from the shoulder has a lesser outer diameter than a portion of the sidewall of the first infusion tube extending proximally from the shoulder. The sidewall of the distal tip defines a bore adapted to receive the portion of the sidewall of the first infusion tube extending distally from the shoulder, such that a portion of the sidewall of the distal tip overlaps the portion of the sidewall of the first infusion tube extending distally from the shoulder.

In some embodiments, the cannula assembly further includes a basket disposed between the sidewall of the distal tip and the sidewall of the first infusion tube, the basket comprising a plurality of individual members arranged to prevent collapse of the distal lumen.

In some embodiments, the basket is comprised of a shape memory material defining a first profile at a first temperature and a second profile at a second temperature.

In some embodiments, the first profile is a straight tubular profile, the first temperature is a temperature at which the distal tip is connected to the distal end of the first elongate body during a manufacturing process of the dual lumen coaxial cannula assembly, the second profile is a tubular structure curving about an axis perpendicular to a longitudinal axis of the first elongate body, and the second temperature is a human body temperature. The basket transitions from the first profile to the second profile as a temperature of the dual lumen coaxial cannula assembly transitions from the first temperature to the second temperature.

In some embodiments, the second profile is adapted for positioning in a bend of a patient's pulmonary artery.

In some embodiments, a profile of the distal tip defines a tubular structure curving about an axis perpendicular to a longitudinal axis of the first elongate body.

In some embodiments, the profile of the distal tip is adapted for positioning in a bend of a patient's pulmonary artery.

In some embodiments, the distal end of the distal tip defines an end feature shaped to reduce the prevalence of edges of the distal tip in order to ease venous insertion of the dual lumen coaxial cannula assembly into the patient and reduce the risk of vessel wall injury.

In some embodiments, the end feature has a profile of at least one of a balloon, a hemisphere, an oval, and a parabola.

In some embodiments, the distal tip further includes a flap movable between a closed position preventing fluid flow out of the distal lumen and an opened position permitting fluid flow out of the distal lumen. In the closed position of the flap, fluid flow into the distal lumen creates backpressure forcing fluid out of one or more infusion apertures in the first elongate body.

In some embodiments, the flap is biased in the closed position and movable to the opened position by application of a force to a distal side of the flap.

Other examples not part of the invention are directed to a method of inserting a cannula assembly into a patient's vasculature. The method includes inserting an introducer into the patient's jugular vein; maneuvering a distal end of the introducer through the patient's right atrium and right ventricle and into the patient's pulmonary artery, the introducer having a length such that a proximal end of the introducer extends out of the patient's body when the distal end of the introducer is positioned in the patient's pulmonary artery; sliding a central lumen of the cannula assembly over the introducer and maneuvering the cannula assembly along the introducer such that a first distal end of the cannula is positioned at least within proximity of the patient's pulmonary artery and such that a second distal end of the cannula is at least within proximity of the patient's right atrium; and removing the introducer from the patient through the central lumen of the cannula assembly. The cannula assembly includes a distal tip connected to the first distal end of the cannula assembly, wherein the distal tip is comprised of a material having a lower hardness than a hardness of the first distal end.

In some examples, as the central lumen of the cannula assembly is slid over the introducer, the introducer deflects a flap in the cannula assembly from a closed position for preventing fluid flow out of a distal end of the central lumen to an opened position. Removing the introducer allows the flap to deflect back to the closed position for preventing fluid flow out of a distal end of the central lumen.

In some examples, after removal of the introducer, the distal tip of the cannula assembly transitions from a first profile defining a straight tubular structure to a second profile defining a tubular structure curved about an axis perpendicular to a longitudinal axis of the cannula assembly.

In some examples, the transition from the first profile to the second profile is caused by a temperature change of a shape memory material forming at least a portion of the distal tip.

In some examples, the temperature change may include a change from a first temperature to a second temperature. The first temperature may be a temperature at which the distal tip is connected to the distal end of the first elongate body during a manufacturing process of the cannula assembly, and the second temperature may be a body temperature of the patient.

Further details and advantages of the present disclosure will be understood from the following detailed description read in conjunction with the accompanying drawings.

### BRIEF DESCRIPTION OF THE DRAWINGS

**FIG. 1** is a top view of one embodiment of a coaxial cannula shown with a connector.
**FIG. 2** is a side view of the coaxial cannula shown in **FIG. 1****.**
**FIG. 3** is a top view of one embodiment of an infusion cannula.
**FIG. 4A** is a cross-sectional view of detail **A** shown in **FIG. 3****,** according to an embodiment of the coaxial cannula.
**FIG. 4B** is a cross-sectional view of detail **A** shown in **FIG. 3****,** according to another embodiment of the coaxial cannula.
**FIG. 4C** is a cross-sectional view of detail **A** shown in **FIG. 3****,** according to another embodiment of the coaxial cannula.
**FIG. 4D** is a cross-sectional view of detail **A** shown in **FIG. 3****,** according to another embodiment of the coaxial cannula.
**FIG. 4E** is a cross-sectional view of detail **A** shown in **FIG. 3****,** according to another embodiment of the coaxial cannula.
**FIG. 4F** is an exploded perspective view of detail **A** shown in **FIG. 3****,** according to another embodiment of the coaxial cannula.
**FIG. 4G** is a cross-sectional view of **FIG. 4F****.**
**FIG. 4H** is a cross-sectional view of detail **A** shown in **FIG. 3****,** according to another embodiment of the coaxial cannula.
**FIG. 5** is a top view of one embodiment of a drainage cannula.
**FIG. 6** is a cross-sectional view of detail **B** in **FIG. 5****.**
**FIG. 7** is a top cross-sectional view of the coaxial cannula taken along line **A-A** in **FIG. 2****.**
**FIG. 8** is a cross-sectional view of detail **C** in **FIG. 7****,** illustrating a transition portion at a distal end of a drainage cannula of the coaxial cannula.
**FIG. 9** is a perspective view of a connector shown coupled to a coaxial cannula according to another embodiment.
**FIG. 10** is a rear perspective view of the coaxial cannula shown in **FIG. 9****.**
**FIG. 11** is a schematic view of one embodiment of a coaxial cannula positioned in the superior vena cava of a body of a patient.
**FIG. 12** is a schematic view of one embodiment of a coaxial cannula positioned inside a patient's heart.
**FIG. 13** is a schematic view of the coaxial cannula of any of **FIGS. 4F-H** positioned inside a patient's heart.

### DETAILED DESCRIPTION

For purposes of the description hereinafter, the terms "end," "upper," "lower," "right," "left," "vertical," "horizontal," "top," "bottom," "lateral," "longitudinal," and derivatives thereof shall relate to the invention as it is oriented in the drawing figures. However, it is to be understood that the invention may assume various alternative variations and step sequences, except where expressly specified to the contrary. It is also to be understood that the specific devices and processes illustrated in the attached drawings, and described in the following specification, are simply exemplary embodiments or aspects. Hence, specific dimensions and other physical characteristics related to the embodiments or aspects disclosed herein are not to be considered as limiting.

As used herein, the term "at least one of" is synonymous with "one or more of". For example, the phrase "at least one of A, B, and C" means any one of A, B, and C, or any combination of any two or more of A, B, and C. For example, "at least one of A, B, and C" includes one or more of A alone; or one or more B alone; or one or more of C alone; or one or more of A and one or more of B; or one or more of A and one or more of C; or one or more of B and one or more of C; or one or more of all of A, B, and C. Similarly, as used herein, the term "at least two of" is synonymous with "two or more of". For example, the phrase "at least two of D, E, and F" means any combination of any two or more of D, E, and F. For example, "at least two of D, E, and F" includes one or more of D and one or more of E; or one or more of D and one or more of F; or one or more of E and one or more of F; or one or more of all of D, E, and F.

When used in relation to a cannula, catheter, or other device inserted into a patient, the term "proximal" refers to a portion of such device farther from the end of the device inserted into the patient. When used in relation to a cannula, catheter, or other device inserted into a patient, the term "distal" refers to a portion of such device nearer to the end of the device inserted into the patient.

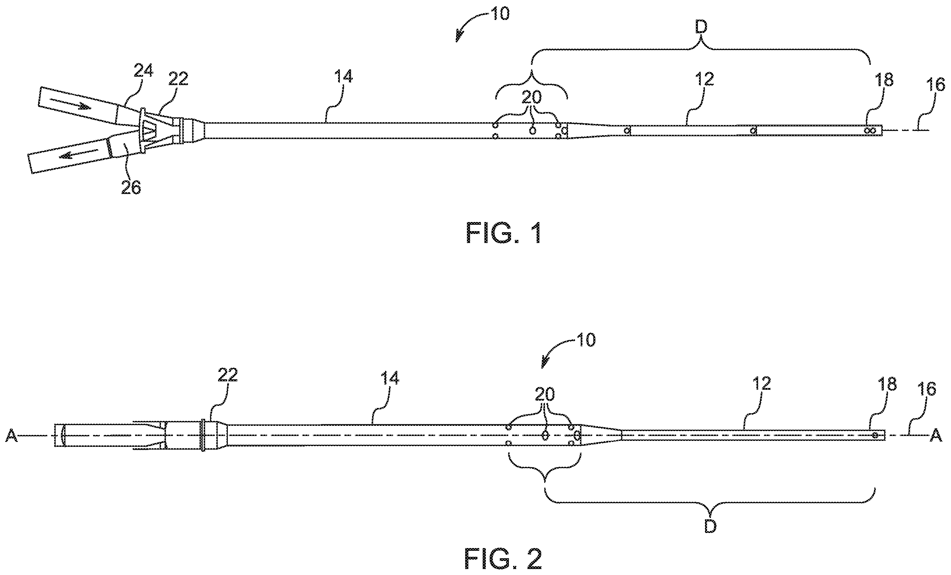

Referring to the drawings, in which like reference characters refer to like parts throughout the several views thereof, various embodiments of a coaxial, dual lumen cannula **10** (hereinafter referred to as "coaxial cannula **10")** are shown. With initial reference to **FIGS. 1-2****,** the assembled coaxial cannula **10,** according to one embodiment, generally includes a first infusion tube **12** having a first length and a second drainage tube **14** having a second length. The first length of the first infusion tube **12** is greater than the second length of the second drainage tube **14.**

The first infusion tube **12** is disposed within the second drainage tube **14** in a coaxial arrangement centered about a central axis **16.** Each of the first infusion tube **12** and the second drainage tube **14** has a first circumference defining a first lumen and a second circumference defining a second lumen, respectively. The first circumference of the first infusion tube **12** is smaller than the second circumference of the second drainage tube **14** such that the first infusion tube **12** may be placed within the second lumen of the second drainage tube **14.** One or both of the first infusion tube **12** and the second drainage tube **14** may be manufactured from a medical-grade material such as polyurethane. Alternatively, the tubes may be made from PVC or silicone, and may be dip molded, extruded, co-molded, or made using any other suitable manufacturing technique.

The coaxial cannula **10** has sufficient placement flexibility adapted for placement of the coaxial cannula **10** within a patient's body. A vascular insertion site is provided at the internal jugular vein on the patient's neck area or other suitable venous location. The coaxial cannula **10** is adapted for placement above or below the right atrium of the patient's heart. The coaxial cannula **10** may be used with an introducer, such as a guide wire, to guide the placement of the coaxial cannula **10** as it is inserted within the patient's body.

With continuing reference to **FIGS. 1** and **2****,** the coaxial cannula **10** is designed to withdraw blood directly from the patient's heart and return blood back into the patient's heart. The function of the first infusion tube **12** is to deliver blood into the bloodstream of the patient, while the function of the second drainage tube **14** is to drain the blood from the patient's bloodstream, as will be described hereafter.

A plurality of infusion apertures **18** is provided at a distal end of the first infusion tube **12.** The plurality of infusion apertures **18** is desirably arranged in a circular pattern extending around the outer circumference of the first infusion tube **12.** In some embodiments, the plurality of infusion apertures **18** may be disposed in multiple groups provided at various sites on the first infusion tube **12.** Similarly, the second drainage tube **14** includes a plurality of drainage apertures **20** provided at a distal end of the second drainage tube **14.** The plurality of drainage apertures **20** is desirably arranged in a circular pattern extending around the outer circumference of the second drainage tube **14.** In alternative embodiments, the plurality of drainage apertures **20** may be arranged in groups disposed at various sites along the length of the second drainage tube **14.** The infusion apertures **18** are separated from the drainage apertures **20** by a distance **D.** In different embodiments of the coaxial cannula **10,** the separation of infusion apertures **18** from drainage apertures **20** determines the amount of mixing of oxygenated blood and unoxygenated blood. This distance may vary based on the age and size of the patient, as well as the desired flow rates during the medical procedure where the coaxial cannula **10** is used. For example, a coaxial cannula **10** having a specific overall length and diameter, along with a desired pattern and distance between the infusion apertures **18** and the drainage apertures **20,** may be selected based on age and/or size of the patient.

With continuing reference to **FIGS. 1** and **2****,** a connector **22** is provided at the proximal end of the coaxial cannula **10.** The connector **22** includes an inlet portion **24** in fluid communication with the first infusion tube **12** to transfer blood from a blood pump (not shown) to the first infusion tube **12.** An outlet portion **26** of the connector **22** is in fluid communication with the second drainage tube **14** to transfer blood from the second drainage tube **14** to the blood pump. The outlet portion **26** and the inlet portion **24** of the connector **22** are arranged such that the fluid pathways leading from the second drainage tube **14** and to the first infusion tube **12** transition from a coaxial arrangement at a distal end of the connector **22** to an axially-offset arrangement at a proximal end of the connector **22.** Details of the connector **22** construction will be discussed in greater detail below.

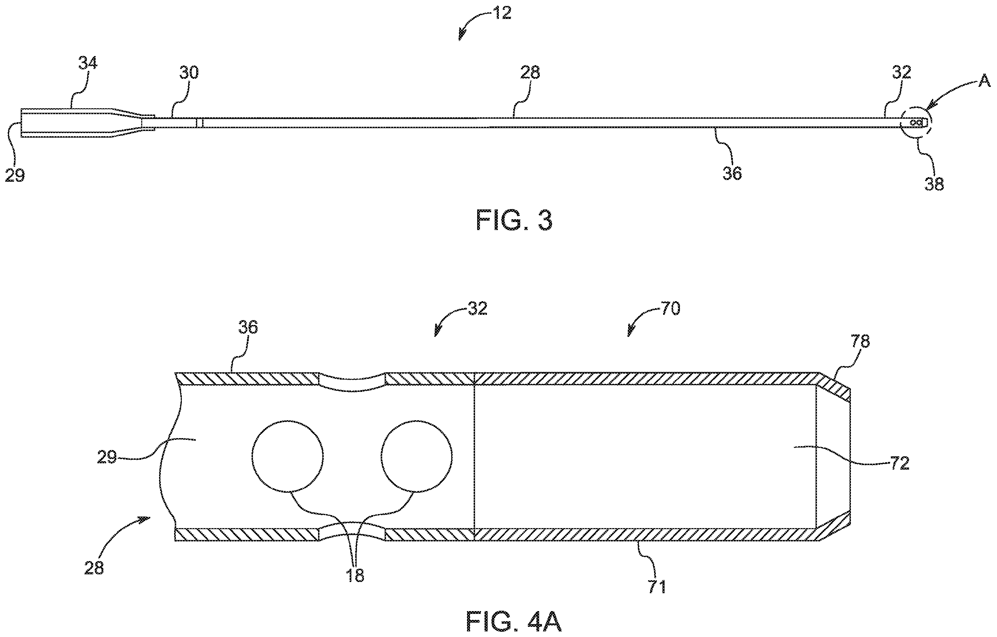

With reference to **FIGS. 3-4G****,** and with continuing reference to **FIGS. 1** and **2****,** the first infusion tube **12** of the coaxial cannula **10** is illustrated. The first infusion tube **12** has a first elongate body **28** having a working length of, for example, around 21 cm. The first elongate body **28** of the first infusion tube **12** is substantially cylindrical and extends from a first proximal end **30** to a first distal end **32.** The first elongate body **28** includes a first lumen **29** extending throughout the entire length of the first infusion tube **12.** The first proximal end **30** includes a first connector portion **34** for coupling the first infusion tube **12** to the inlet portion **24** of the connector **22.** The first elongate body **28** of the first infusion tube **12** has a hollow structure defined by a first sidewall **36** extending circumferentially about the first elongate body **28.** The first sidewall **36** has a substantially constant thickness throughout the length of the first elongate body **28.** The first distal end **32** of the first elongate body **28** may be attached to a pliable distal tip **70** having a hollow structure defined by a tip sidewall **71.** The distal tip **70** defines a tip lumen **72** in fluid communication with the first lumen **29** of the first elongate body **28** and allowing fluid flow between the tip lumen **72** and the first lumen **29** of the first elongate body **28.** A distal end of the distal tip **70** may include a first tapering section **78** for enabling easier insertion of the first infusion tube **12** into the patient's body.

The distal tip **70** may be made from a medical grade material such as polyurethane, or another material suitable for bonding to the material of the first elongate body **28.** In some embodiments, the distal tip **70** is manufactured from a material having a Shore durometer hardness of less than or equal to 85A to prevent injury and/or discomfort to the patient when the cannula **10** is placed into the patient. In some embodiments, the distal tip **70** may have a Shore durometer hardness of at least 5A less than the Shore durometer hardness of the first elongate body **28.**

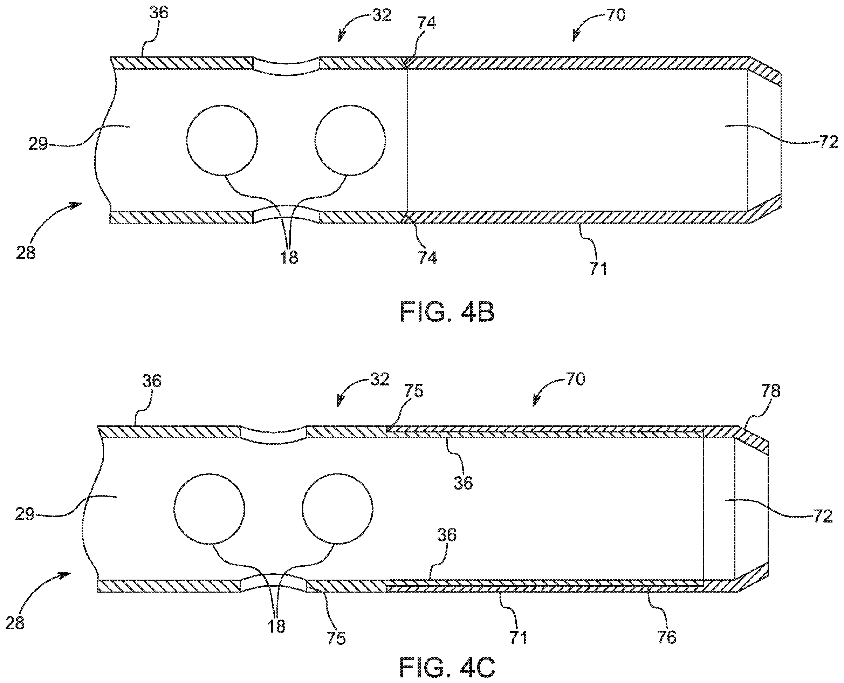

With reference to **FIGS. 4A-4H****,** various embodiments, as well as examples not part of the invention, of the first distal end **32** of the first elongate body **28** and the distal tip **70** are shown. In one example shown in **FIG. 4A****,** the first sidewall **36** of the first elongate body **28** and the tip sidewall **71** of the distal tip **70** abut one another on a plane substantially perpendicular to the longitudinal axis of the first elongate body **28.** The distal tip **70** may be fused to the first elongate body **28** via a reflow process utilizing heated glass molds or a heat-shrinkable material such as polyethylene terephthalate (PET). Alternatively, the distal tip **70** may be fused to the first elongate body **28** via radiofrequency welding. One or both of the distal tip **70** and the first elongate body **28** may be subjected to a buffing operation prior to joining in order to increase surface area of the connection between the distal tip **70** and the first elongate body **28,** thereby increasing resistance to tensile separation of the distal tip **70** and the first elongate body **28.** The embodiment shown in **FIG. 4B** is substantially identical to the example shown in **FIG. 4A****,** except that the distal tip **70** includes a connecting taper **74** interacting with a respective taper of the first elongate body **28** to further increase the surface area of the connection between the distal tip **70** and the first elongate body **28.** The respective tapers of the distal tip **70** and the first elongate body **28** also assist in aligning the tip lumen **72** and the first lumen **29** of the first elongate body **28** during the fusing of the distal tip **70** and the first elongate body **28.**

In the claimed embodiment shown in **FIG. 4C****,** the first elongate body **28** includes a shoulder **75** reducing the outer diameter of the first sidewall **36.** The shoulder **75** may be formed by a grinding operation after the first elongate body **28** is molded or otherwise formed. In some embodiments, the distance from the shoulder **75** to the first distal end **32** of the first intrusion tube **12** may be approximately 5 centimeters. The thickness of the first sidewall **36** may be reduced by at least 50% at the shoulder **75.** In some embodiments, the first sidewall **36** may have the same outer diameter from the shoulder **75** to the first distal end **32.** In other embodiments, the outer diameter of the first sidewall **36** may taper from the shoulder **75** to the first distal end **32.** The distal tip **70** has a bore **76** sized to slide over and interface with the reduced outer diameter of the first sidewall **36** and abut the shoulder **75,** forming a lap joint between the distal tip **70** and the first elongate body **28.** The interfacing external surface of the first sidewall **36** and the internal surface of the bore **76** of the distal tip **70** may be joined via a reflow process or radiofrequency welding as discussed with reference to the embodiment of **FIG. 4A****.**

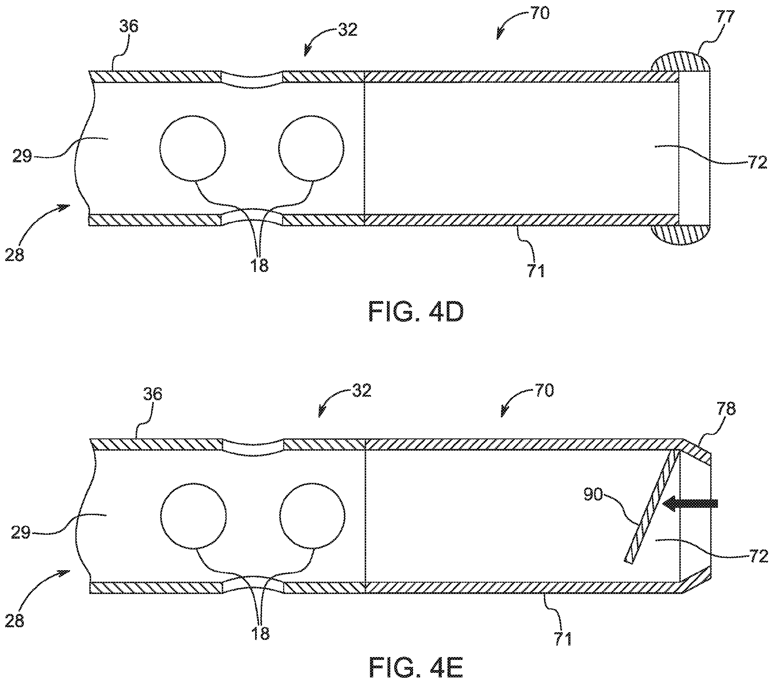

In one embodiment shown in **FIG. 4D****,** the distal tip **70** may include an end feature such as a balloon **77** to ease insertion of the coaxial cannula **10** into the patient and further reduce patient discomfort and risk of vessel wall injury by eliminating sharp edges of the distal tip **70.** Profiles of end features other than the balloon 77 which eliminate or mitigate sharp edges of the distal tip **70** may be appreciated by one skilled in the art. For example, the end feature may have a hemispherical, ovaline, teardrop, or parabolic profile.

In one embodiment shown in **FIG. 4E****,** the distal tip **70** may include a flap **90** closable by fluid pressure to prevent fluid flow out of the tip lumen **72.** Fluid flowing from the proximal end of the first elongate body **28** to the distal end of the first elongate body **28** applies pressure to a proximal side of the flap **90,** biasing the flap **90** to a closed position and creating back pressure against fluid flow out of the infusion apertures **18.** The flap **90** may be deflected to an opened position by application of a force to the distal side of the flap **90.** For example, during insertion of the coaxial cannula **10** into the patient, the flap **90** may be moved to the opened position by the introducer as the coaxial cannula **10** is slid over the distal end of the introducer.

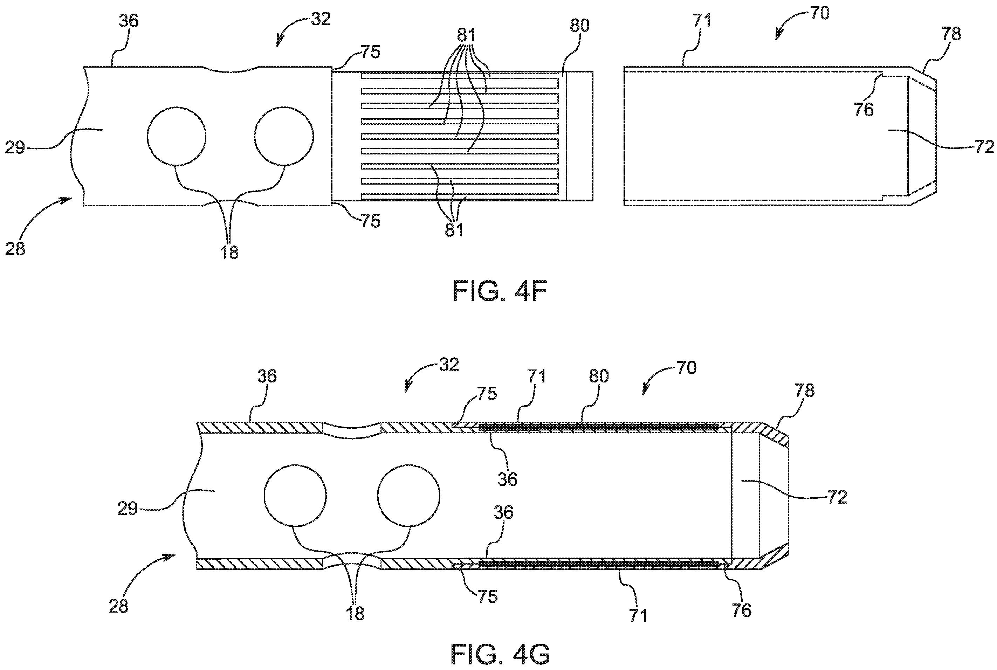

In one embodiment shown in **FIGS. 4F-4G****,** a basket **80** may be disposed between the distal tip **70** and the first sidewall **36** to resist collapse of the tip lumen **72** due to forces on the tip sidewall **71.** The basket **80** includes a plurality of individual members **81** extending over the outer surface of the first sidewall **36** to provide hoop strength to the first sidewall **36.** The arrangement of the individual members **81** may form any design balancing improved hoop strength with pliability. In general, covering a greater area of the outer surface of the first sidewall **36** with the individual members **81** results in greater hoop strength but less pliability, and vice versa. However, one skilled in the art may appreciate and utilize various geometric arrangements of the individual members **81** that optimize hoop strength and pliability. Suitable materials for manufacturing the basket **80** include biocompatible metal or metal alloys such as stainless steel or nickel titanium alloy (nitinol). The individual members **81** may be formed via chemical or laser etching or other suitable processes. The basket **80** may be joined to the first sidewall **36** via an adhesive, such as biocompatible or medical-grade Loctite^{®}, prior to joining the distal tip **70** to the first sidewall **36.** The distal tip **70,** which in this embodiment is designed such that the bore **76** fits over the basket **80,** may then be joined to the sidewall **36** via a reflow process or radiofrequency welding as discussed with reference to the examples of **FIGS. 4A** and **4C****.**

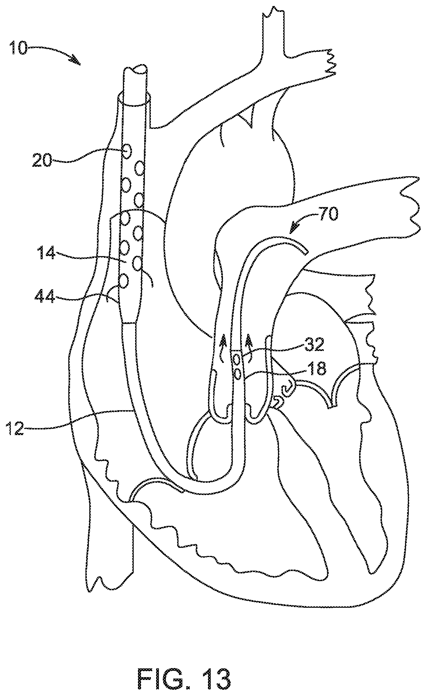

In some embodiments, the basket **80** may be manufactured of a shape memory material, such as nitinol, such that the basket **80** defines a first profile at a first temperature and a second profile at a second temperature. For example, the first profile of the basket **80** may be a substantially straight tubular structure as shown in **FIG. 4F****.** The first profile may be exhibited at a manufacturing temperature at which the basket **80** and the distal tip **70** are joined to the first elongate body **28.** The second profile of the basket **80** may be a curved tubular structure or pigtail, as shown in **FIG. 13****.** Specifically, the second profile may be a tubular structure curved about an axis perpendicular to a longitudinal axis of the cannula assembly **10.** The second profile may be exhibited at human body temperature, particular the body temperature within the pulmonary artery, for example 98.6° Fahrenheit. In such an embodiment, the straight first profile of the basket **80** facilitates assembly of the first infusion tube **12.** As the first infusion tube **12** is inserted into the patient, the basket **80** and the distal tip **70** adhered thereto transitions to the curved second profile to facilitate placement of the distal tip **70** in the patient's pulmonary artery.

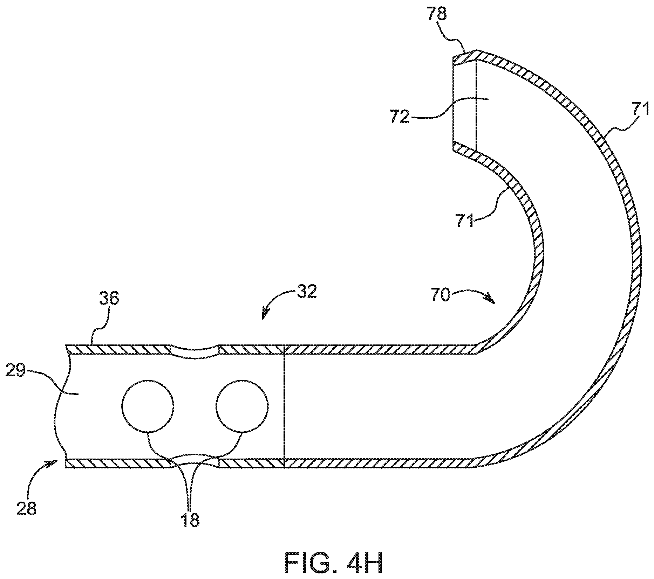

In one embodiment shown in **FIG. 4H****,** the distal tip **70** may be pre-formed into a curved or pigtail profile. The distal tip **70** may be joined to the first elongate body **28** in any suitable manner described with reference to **FIGS. 4A-4G****.** As noted with reference to **FIG. 13****,** the curved or pigtail profile facilitates placement and retention of the distal tip **70** in the patient's pulmonary artery. Additionally, the distal tip **70** of the embodiment of **FIG. 13** may be made of a particularly soft material relative to the first infusion tube **12** to prevent damage to the arterial walls of the patient. The tip lumen **72** of the distal tip **70** may be closed in some embodiments where the distal tip **70** is used only for retention in the pulmonary artery and not to deliver blood to the pulmonary artery.

With continued reference to **FIGS. 4A-4H****,** the plurality of infusion apertures **18** is provided at the first distal end **32** of the first infusion tube **12.** The plurality of infusion apertures **18** extends circumferentially around the first distal end **32.** Each infusion aperture **18** has a diameter of, for example, about 1 mm. The plurality of infusion apertures **18** may be arranged in an alternating pattern of axially offset rows of infusion apertures **18** arranged around the circumference of the first infusion tube **12.** Each of the plurality of infusion apertures **18** extends through the thickness of the first sidewall **36.** The infusion apertures **18** illustrated in **FIGS. 3** and **4** extend through the first sidewall **36** in a direction perpendicular to a longitudinal axis of the first elongate body **28.** Alternatively, the plurality of infusion apertures **18** may extend through the thickness of the first sidewall **36** in an angled manner with respect to the longitudinal axis of the first elongate body **28.** For example, the plurality of infusion apertures **18** may be arranged at an acute or obtuse angle with respect to a cross-sectional plane of the first infusion tube **12** extending perpendicular to the longitudinal axis of the first elongate body **28.** In some embodiments, a wire mesh basket is provided inside the first lumen **29** of the first infusion tube **12** at a location surrounding the infusion apertures **18.** The wire mesh basket supports and prevents the first sidewall **36** from collapsing due to being weakened by the formation of the plurality of infusion apertures **18.** In one embodiment, one or more sensors (not shown) may be provided at the first distal end **32** of the first infusion tube **12.** The sensor(s) may be adapted for measuring, for example, local blood pressure and/or oxygen concentration.

The total cross-sectional area of the plurality of infusion apertures **18** is desirably approximately equal to or greater than the cross-sectional area of the first lumen **29.** If the cross-sectional area of the plurality of infusion apertures **18** is less than the cross-sectional area of the first lumen **29,** an undesirable pressure drop may occur. This pressure drop reduces the flow throughput within the first lumen **29** and impairs the efficiency of the first infusion tube **12.** Desirably, the total cross-sectional area of the plurality of infusion apertures **18** exceeds the cross-sectional area of the first lumen **29** such that if one or more of the infusion apertures **18** becomes clogged, the total cross-sectional area of the remaining infusion apertures **18** is equal to or greater than the cross-sectional area of the first lumen **29.** In this manner, the blood flow through the first lumen **29** is maximized even if one or more of the infusion apertures **18** becomes clogged.

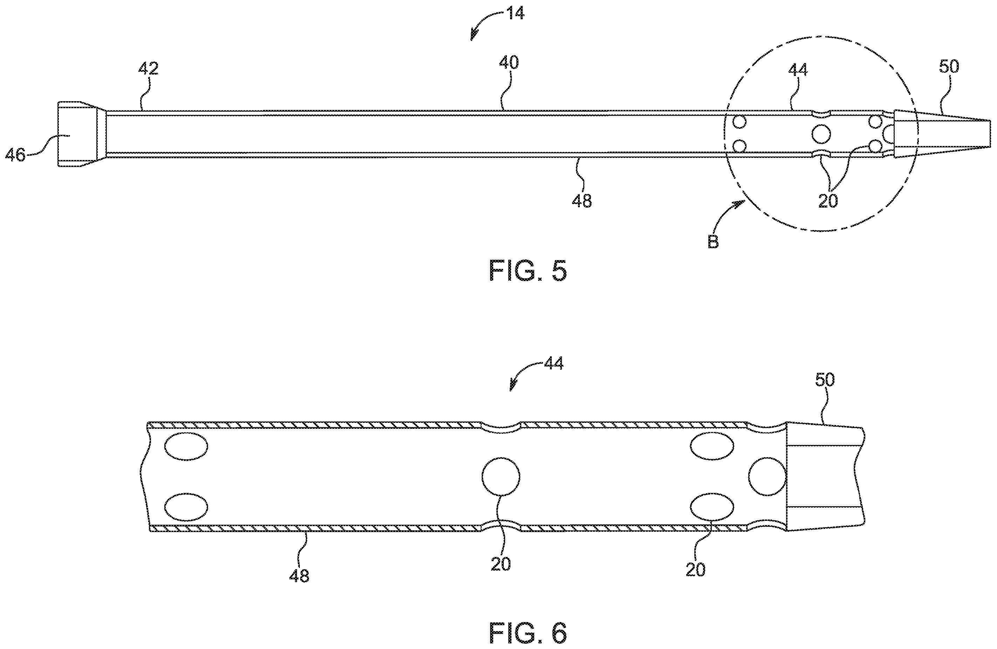

With reference to **FIGS. 5-6****,** and with continuing reference to **FIGS. 1** and **2****,** the second drainage tube **14** is illustrated separately from the coaxial cannula **10.** The second drainage tube **14** has a second elongate body **40** having a working length of, for example, around 12 cm. The second elongate body **40** of the second drainage tube **14** is substantially cylindrical and extends from a second proximal end **42** to a second distal end **44.** The second elongate body **40** includes a second lumen **46** extending throughout the entire length of the second drainage tube **14.** The second proximal end **42** includes a second connector portion **47** for coupling the second drainage tube **14** to the outlet portion **26** of the connector **22.** The second elongate body **40** of the second drainage tube **14** has a hollow structure defined by a second sidewall **48** extending circumferentially about the second elongate body **40.** The second sidewall **48** has a substantially constant thickness throughout the length of the second elongate body **40,** with a second tapering section **50** at the second distal end **44** of the second elongate body **40.** At the second proximal end **42** of the second elongate body **40,** the second sidewall **48** gradually increases in thickness before transitioning into the second connector portion **47.** The second tapering section **50** located at the second distal end **44** has a thinner second sidewall **48** but retains the internal diameter of the second lumen **46.** The second tapering section **50** enables easier insertion of the second drainage tube **14** into the patient's body.

With specific reference to **FIG. 6****,** the second distal end **44** of the second drainage tube **14** is shown. The plurality of drainage apertures **20** is provided at the second distal end **44** of the second drainage tube **14.** The plurality of drainage apertures **20** extends circumferentially around the second distal end **44.** Each drainage aperture **20** has a diameter of, for example, about 1.5 mm. The plurality of drainage apertures **20** may be arranged in an alternating pattern of axially offset rows around the circumference of the second drainage tube **14.** Each of the plurality of drainage apertures **20** extends through the thickness of the second sidewall **48.** The drainage apertures illustrated in **FIGS. 5** and **6** extend through the second sidewall **48** in a direction perpendicular to a longitudinal axis of the second elongate body **40.** Alternatively, the plurality of drainage apertures **20** may extend through the thickness of the second sidewall **48** in an angled manner with respect to the longitudinal axis of the second elongate body **40.** For example, the plurality of drainage apertures **20** may be arranged at an acute or obtuse angle with respect to a cross-sectional plane of the second drainage tube **14** extending perpendicular to the longitudinal axis of the second elongate body **40.** In some embodiments, a wire mesh basket (not shown in **FIG. 6****)** is provided inside the second lumen **46** of the second drainage tube **14** at a location surrounding the drainage apertures **20.** The wire mesh basket supports and prevents the second sidewall **48** from collapsing due to being weakened by the formation of the plurality of drainage apertures **20.** In one embodiment, one or more sensors (not shown) may be provided at the second distal end **44** of the second drainage tube **14.** The sensor(s) may be adapted for measuring, for example, local blood pressure and/or oxygen concentration.

The total cross-sectional area of the plurality of drainage apertures **20** is desirably approximately equal to or greater than the cross-sectional area of the second lumen **46.** If the cross-sectional area of the plurality of drainage apertures **20** is less than the cross-sectional area of the second lumen **46,** an undesirable pressure drop within the second drainage tube **14** may occur. This pressure drop reduces the flow throughput within the second lumen **46** and impairs the efficiency of the second drainage tube **14.** Desirably, the total cross-sectional area of the plurality of drainage apertures **20** exceeds the cross-sectional area of the second lumen **46** such that if one or more drainage apertures **20** becomes clogged, the total cross-sectional area of the remaining drainage apertures **20** is equal to or greater than the cross-sectional area of the second lumen **46.** In this manner, the blood flow through the second lumen **46** is maximized even if one or more of the drainage apertures **20** becomes clogged.

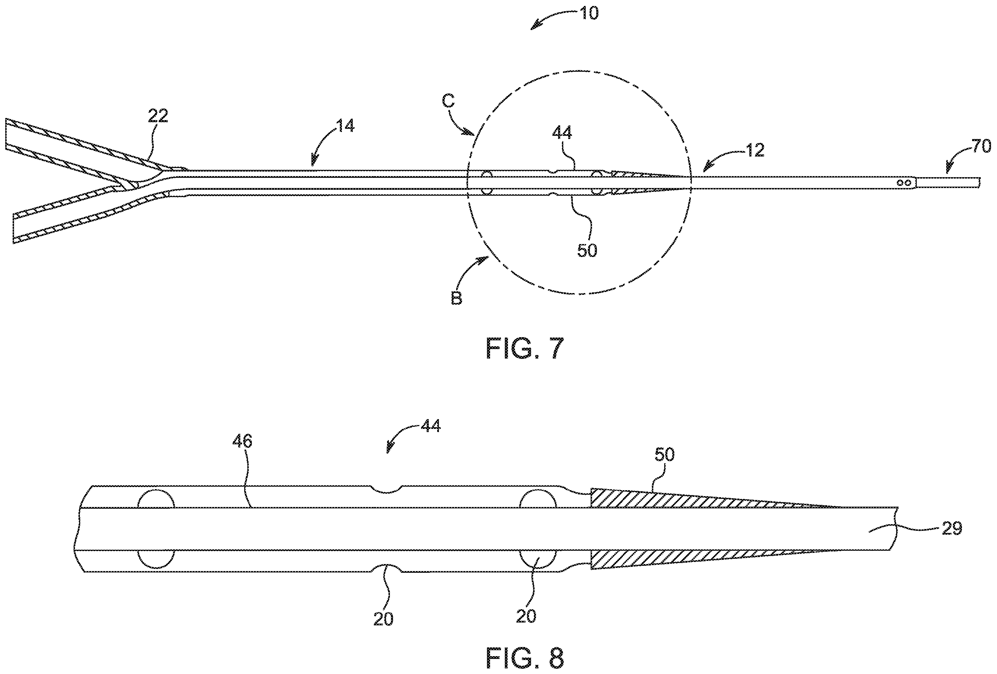

With reference to **FIG. 7****,** the coaxial cannula **10** shown in **FIGS. 1** and **2** is illustrated in cross section. The second distal end **44** of the second drainage tube **14** is fixedly attached to a mid portion of the first infusion tube **12** along the length of a second tapering section **50,** as shown in **FIG. 8****.** The first infusion tube **12** and the second drainage tube **14** are coupled to the connector **22** in such manner that the first infusion tube **12** and the second drainage tube **14** cross inside the connector **22** body without being connected to each other.

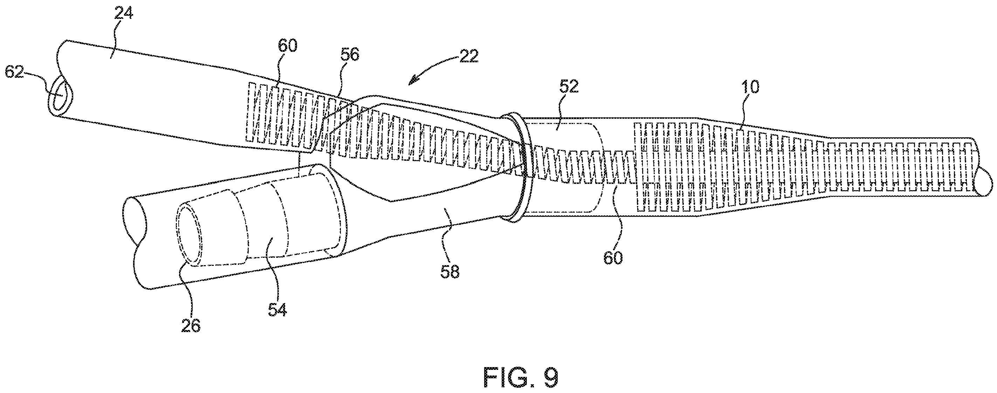

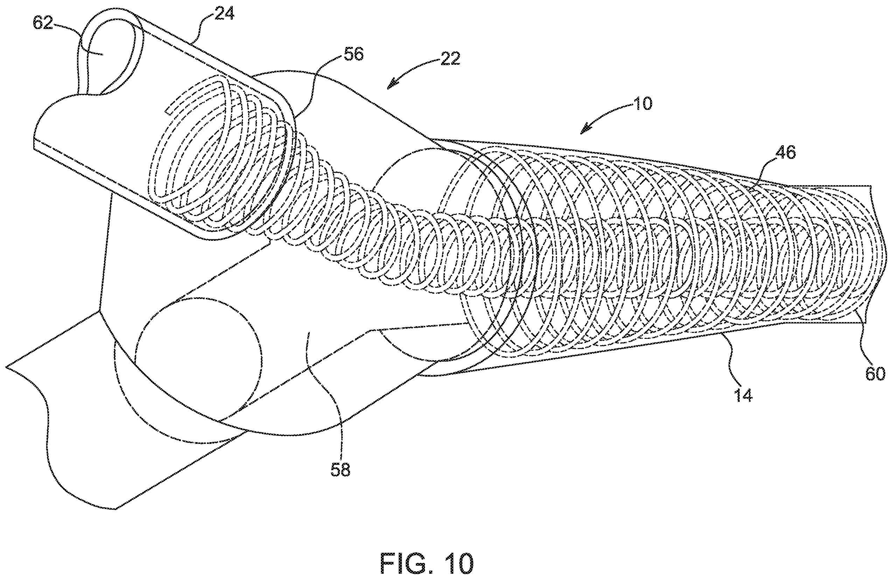

With reference to **FIGS. 9** and **10****,** a detailed view of the connector **22** is shown coupled to a coaxial cannula **10** according to another embodiment. **FIG. 9** illustrates the connector **22** showing the fluid pathways extending through the interior of the connector **22.** The connector includes a distal aperture **52** at a distal end of the connector **22** for connecting to the proximal end of the coaxial cannula **10.** The proximal end of the connector **22** has the inlet portion **24** and the outlet portion **26** in fluid communication with the distal aperture **52.** The outlet portion **26** may include a barbed fitting **54** for connecting a drainage connection **57** that extends to a blood pump. The inlet portion **24** includes an inner tube **56** that extends through the interior of the connector **22** and connects with the first infusion tube **12.** The inner tube **56** may extend beyond the distal aperture **52** for connecting with the first infusion tube **12.** A drainage opening **58** connects the second drainage tube **14** with the outlet portion **26** of the connector **22.** The drainage opening **58** is coextensive with the inner tube **56** along the length of the body portion of the connector **22.** The inner tube **56** may be reinforced with a metal or plastic coil **60** that extends in a helical manner along the length of the inner tube **56** to minimize kinking and/or collapse of the first infusion tube **12.**

With continuing reference to **FIGS. 9** and **10****,** the inner tube **56** passes through the connector **22** to provide a smooth, seamless transition from a single distal aperture **52** to the branched arrangement of the inlet portion **24** and the outlet portion **26.** The transition is desirably void of any j oints, welds, and/or other connections that can create irregularities in the flow of blood and can damage blood cells.

In use, the proximal end of the coaxial cannula **10** is connected to the distal aperture **52** of the connector **22.** The inner tube **56** receives blood from a supply line **62** and sends it through the lumen of the inner tube **56** to the first infusion tube **12.** As the diameter of the inner tube **56** is smaller than the diameter of the drainage opening **58,** the inner tube **56** extends through the interior of the connector **22,** thus allowing the inner tube **56** to be continuous throughout the length of the connector **22.** Depending on the application, the inner tube **56** may or may not include structural reinforcement in the form of the coil **60.** In embodiments in which the inner tube **56** is reinforced with the coil **60,** the inner tube **56** is stronger and less susceptible to kinking or collapse.

The connector **22** may be made from polycarbonate as an example, but could also be made from PVC, acrylic, or polyurethane. The connector **22** may be constructed using one or more manufacturing techniques including injection molding, machining, or dip forming. One of ordinary skill in the art will understand that a variety of other manufacturing techniques may be used for constructing the connector **22** without departing from the intended scope of the invention.

With continued reference to **FIGS. 9** and **10****,** one or both of the first elongate body **28** of the first infusion tube **12** and the second elongate body **40** of the second drainage tube **14** includes a helical coil **60** extending through the length thereof. The helical coil **60** may be disposed along the interior surface of the first lumen **29** and the second lumen **46.** Alternatively, the helical coil **60** may be disposed within the first sidewall **36** and the second sidewall **48.** The helical coil **60** may be manufactured from medical-grade metal or plastic.

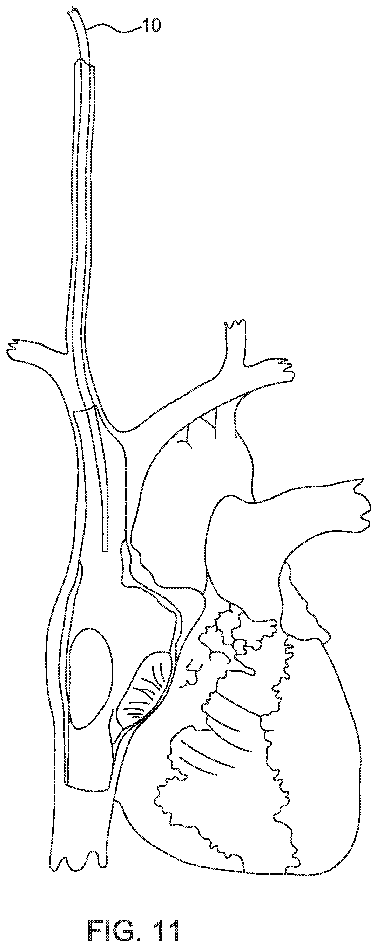

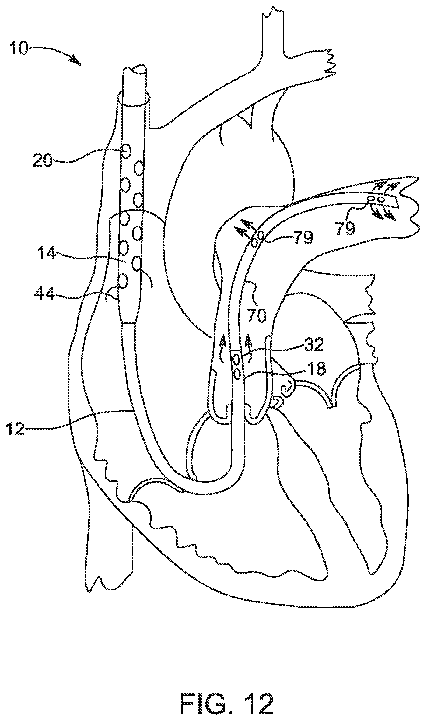

Having described several non-limiting embodiments and examples of the coaxial cannula **10** and the connector **22,** an exemplary and non-limiting method for supporting the right heart of a patient using the coaxial cannula **10** and the connector **22** will now be described with reference to **FIG. 11****, albeit not forming part of the invention**. In use, the coaxial cannula **10** is inserted into the pulmonary artery (PA) in a percutaneous procedure. The coaxial cannula **10** withdraws blood from the patient's heart and delivers blood back to the patient. Initially, a percutaneous entry needle (not shown) is used to access the patient's internal jugular vein (IJV). An introducer, such as a guidewire, is then inserted through the needle until the tip of the introducer is positioned in the upper portion of the inferior vena cava/right atrium (IVC/RA) junction. The needle can then be removed and a pulmonary wedge catheter inserted over the guidewire into the PA. The introducer tip is then threaded into the PA, and the wedge catheter is removed. The IJV is then serially dilated and the coaxial cannula **10** is threaded along the introducer into the IJV, through the right ventricle, and into the PA. The distal tip **70** of the first infusion tube **12** is sufficiently flexible as to allow it to be easily flexed about the longitudinal axis of the first elongate body **28** to navigate the IJV, right ventricle, and PA. The coaxial cannula **10** may include insertion depth markers and radiopaque markers for aiding the user in placing the coaxial cannula **10** in the right atrium. Once the position of the coaxial cannula **10** is acceptable, the introducer may be removed and the coaxial cannula **10** may clamped in place. For example, the coaxial cannula **10** may be secured to the patient's neck using a suture. **FIG. 12** shows the coaxial cannula **10** positioned in the patient according to some embodiments of the disclosure. In particular, the second distal end **44** is positioned at least within proximity with the right atrium, while the first distal end **32** and the distal tip **70** extend through the main pulmonary artery and at least into proximity with either the left or right branch of the pulmonary branch of the pulmonary artery. The coaxial cannula **10** is inserted into the patient such that the infusion apertures **18** discharge blood into the main pulmonary artery. Additionally, the distal tip **70** may be provided with tip infusion apertures **79** to direct additional blood into the left and right branches of the pulmonary artery.

In the embodiment of the coaxial cannula **10** shown and described with reference to **FIG. 4E****,** initial contact of the flap **90** with the distal end of the introducer causes the flap **90** to deflect to the opened position, permitting threading of the coaxial cannula **10** along the introducer. Removal of the introducer permits the flap **90** to bias to the closed position.

**FIG. 13** illustrates the coaxial cannula **10** according to the embodiments of **FIGS. 4F-4H** positioned in the patient. Similarly to **FIG. 12****,** the second distal end **44** is positioned at least within proximity with the right atrium, while the first distal end **32** and the distal tip **70** extend through the main pulmonary artery and at least into proximity with either the left or right branch of the pulmonary branch of the pulmonary artery. The distal tip **70** is curved to better position and retain the distal tip **70** within the pulmonary artery, decreasing patient discomfort and risk of vessel wall damage. In the case of the embodiment of the coaxial cannula **10** according to **FIGS. 4F-4G****,** in which a shape memory alloy basket **80** is utilized with the distal tip **70,** the distal tip **70** transitions to the curved profile shown in **FIG. 13** as the temperature of the coaxial cannula **10** rises to match the patient's body temperature.

While several embodiments of a coaxial cannula are shown in the accompanying figures and described hereinabove in detail, other embodiments will be apparent to, and readily made by, those skilled in the art without departing from the scope of the invention. For example, it is to be understood that this disclosure contemplates, to the extent possible, that one or more features of any embodiment can be combined with one or more features of any other embodiment. Accordingly, the foregoing description is intended to be illustrative rather than restrictive The invention is defined by the features of the appended claims.

## Claims

1. A dual lumen coaxial cannula assembly (10) comprising:
a first infusion tube (12) having a first elongate body (28) defining a first lumen (29) therethrough, the first infusion tube (12) having a proximal end (30), a distal end (32), a first sidewall (36) extending circumferentially therebetween, and a plurality of infusion apertures (18) extending through the first sidewall (36) at the distal end (32) of the first infusion tube (12);
a second drainage tube (14) co-axially aligned with the first infusion tube (12) and having a second elongate body (40) with a second lumen (46) defined by a space between the first infusion tube (12) and the second drainage tube (14), the second drainage tube (14) having a proximal end (42), a distal end (44), a sidewall (48) extending circumferentially therebetween, and a plurality of drainage apertures (20) at the distal end (44) of the second drainage tube (14); and
a connector (22) attached to the proximal end (30) of the first infusion tube (12) and the proximal end (42) on the second drainage tube (14);
wherein the dual lumen coaxial cannula assembly further comprises: a distal tip (70) defining a distal lumen (72) therethrough, **characterized in** the distal tip (70) having a proximal end, a distal end, and a sidewall (71) extending circumferentially therebetween, the distal tip (70) connected to the distal end (32) of the first elongate body (28), wherein the distal tip (70) is comprised of a material having a lower hardness than a hardness of the first elongate body (28); and
wherein the first sidewall (36) of the first infusion tube (12) defines a shoulder (75) such that a portion of the first sidewall (36) of the first infusion tube (12) extending distally from the shoulder (75) has a smaller outer diameter than a portion of the first sidewall (36) of the first infusion tube (36) extending proximally from the shoulder (75), and the sidewall (71) of the distal tip (70) defines a bore (76) adapted to receive the portion of the first sidewall (36) of the first infusion tube (12) extending distally from the shoulder (75), such that a portion of the sidewall (71) of the distal tip (70) overlaps the portion of the first sidewall (36) of the first infusion tube (12) extending distally from the shoulder (75).

2. The dual lumen coaxial cannula assembly (10) of claim 1, further comprising a basket (80) disposed between the sidewall (71) of the distal tip (70) and the first sidewall (36) of the first infusion tube (12), the basket (80) comprising a plurality of individual members (81) arranged to prevent collapse of the distal lumen.

3. The dual lumen coaxial cannula assembly (10) of claim 2, wherein the basket (80) is comprised of a shape memory material defining a first profile at a first temperature and a second profile at a second temperature.

4. The dual lumen coaxial cannula assembly (10) of claim 3, wherein the first profile is a straight tubular profile;
wherein the first temperature is a temperature at which the distal tip (70) is connected to the distal end (32) of the first elongate body (28) during a manufacturing process of the dual lumen coaxial cannula assembly (10);
wherein the second profile is a tubular structure curving about an axis perpendicular to a longitudinal axis of the first elongate body (28);
wherein the second temperature is a human body temperature; and
wherein the basket (80) transitions from the first profile to the second profile as a temperature of the dual lumen coaxial cannula assembly (10) transitions from the first temperature to the second temperature.

5. The dual lumen coaxial cannula assembly (10) of claim 3, wherein the second profile is adapted for positioning in a bend of a patient's pulmonary artery.

6. The dual lumen coaxial cannula assembly (10) of claim 1, wherein a profile of the distal tip (70) defines a tubular structure curving about an axis perpendicular to a longitudinal axis of the first elongate body (28).

7. The dual lumen coaxial cannula assembly (10) of claim 6, wherein the profile of the distal tip (70) is adapted for positioning in a bend of a patient's pulmonary artery.

8. The dual lumen coaxial cannula assembly (10) of claim 1, wherein the distal end of the distal tip (70) defines an end feature shaped to reduce the prevalence of edges of the distal tip (70) in order to ease venous insertion of the dual lumen coaxial cannula assembly into the patient and reduce the risk of vessel wall injury.

9. The dual lumen coaxial cannula assembly (10) of claim 8, wherein the end feature has a profile of at least one of a balloon (77), a hemisphere, an oval, and a parabola.

10. The dual lumen coaxial cannula assembly (10) of claim 1, wherein the distal tip (70) further comprises a flap (90) movable between a closed position preventing fluid flow out of the distal lumen and an opened position permitting fluid flow out of the distal lumen; and wherein, in the closed position of the flap (90), fluid flow into the distal lumen creates backpressure forcing fluid out of one or more of the plurality of infusion apertures (18) in the first elongate body (28).

11. The dual lumen coaxial cannula assembly (10) of claim 10, wherein the flap (90) is biased in the closed position and movable to the opened position by application of a force to a distal side of the flap (90).

## Patentansprüche

1. Koaxiale Doppellumenkanülenanordnung (10), umfassend:
einen ersten Infusionsschlauch (12) mit einem ersten länglichen Körper (28), der ein erstes Lumen (29) dort hindurch definiert, wobei der erste Infusionsschlauch (12) ein proximales Ende (30), ein distales Ende (32), eine erste Seitenwand (36), die sich im Umfang dazwischen erstreckt, und eine Vielzahl von Infusionsöffnungen (18) aufweist, die sich an dem distalen Ende (32) des ersten Infusionsschlauchs (12) durch die erste Seitenwand (36) erstrecken;
einen zweiten Drainageschlauch (14), der koaxial mit dem ersten Infusionsschlauch (12) ausgerichtet ist und einen zweiten länglichen Körper (40) mit einem zweiten Lumen (46), das durch einen Raum zwischen dem ersten Infusionsschlauch (12) und dem zweiten Drainageschlauch (14) definiert ist, aufweist, wobei der zweite Drainageschlauch (14) ein proximales Ende (42), ein distales Ende (44), eine Seitenwand (48), die sich im Umfang dazwischen erstreckt, sowie eine Vielzahl von Drainageöffnungen (20) an dem distalen Ende (44) des zweiten Drainageschlauchs (14) aufweist; und
einen Konnektor (22), der an dem proximalen Ende (30) des ersten Infusionsschlauchs (12) und dem proximalen Ende (42) auf dem zweiten Drainageschlauch (14) befestigt ist;
wobei die koaxiale Doppellumenkanülenanordnung des Weiteren umfasst:
eine distale Spitze (70), die ein distales Lumen (72) dort hindurch definiert, **dadurch gekennzeichnet, dass** die distale Spitze (70) ein proximales Ende, ein distales Ende und eine Seitenwand (71) aufweist, die sich im Umfang dazwischen erstreckt, wobei die distale Spitze (70) mit dem distalen Ende (32) des ersten länglichen Körpers (28) verbunden ist, wobei die distale Spitze (70) aus einem Material mit einer geringeren Härte als einer Härte des ersten länglichen Körpers (28) zusammengesetzt ist; und
wobei die erste Seitenwand (36) des ersten Infusionsschlauchs (12) eine Schulter (75) definiert, so dass ein Abschnitt der ersten Seitenwand (36) des ersten Infusionsschlauchs (12), der sich distal von der Schulter (75) erstreckt, einen kleineren Außendurchmesser als ein Abschnitt der ersten Seitenwand (36) des ersten Infusionsschlauchs (36) hat, der sich proximal von der Schulter (75) erstreckt, und wobei die Seitenwand (71) der distalen Spitze (70) eine Bohrung (76) definiert, die zum Aufnehmen des Abschnitts der ersten Seitenwand (36) des ersten Infusionsschlauchs (12), welcher sich distal von der Schulter (75) erstreckt, eingerichtet ist, so dass ein Abschnitt der Seitenwand (71) der distalen Spitze (70) den Abschnitt der ersten Seitenwand (36) des ersten Infusionsschlauchs (12) überlappt, der sich distal von der Schulter (75) erstreckt.

2. Koaxiale Doppellumenkanülenanordnung (10) nach Anspruch 1, des Weiteren umfassend einen Korb (80), der zwischen der Seitenwand (71) der distalen Spitze (70) und der ersten Seitenwand (36) des ersten Infusionsschlauchs (12) angeordnet ist, wobei der Korb (80) eine Vielzahl von individuellen Elementen (81) umfasst, die angeordnet sind, um Zusammenfallen des distalen Lumens zu verhindern.

3. Koaxiale Doppellumenkanülenanordnung (10) nach Anspruch 2, wobei der Korb (80) aus einem Formgedächtnismaterial zusammengesetzt ist, das bei einer ersten Temperatur ein erstes Profil und bei einer zweiten Temperatur ein zweites Profil definiert.

4. Koaxiale Doppellumenkanülenanordnung (10) nach Anspruch 3, wobei das erste Profil ein gerades rohrförmiges Profil ist;
wobei die erste Temperatur eine Temperatur ist, bei der die distale Spitze (70) während eines Fertigungsprozesses der koaxialen Doppellumenkanülenanordnung (10) mit dem distalen Ende (32) des ersten länglichen Körpers (28) verbunden wird;
wobei das zweite Profil eine rohrförmige Struktur ist, die um eine Achse gekrümmt ist, welche rechtwinklig zu einer Längsachse des ersten länglichen Körpers (28) ist;
wobei die zweite Temperatur eine Temperatur eines menschlichen Körpers ist; und
wobei der Korb (80) von dem ersten Profil zu dem zweiten Profil übergeht, wenn eine Temperatur der koaxialen Doppellumenkanülenanordnung (10) von der ersten Temperatur zu der zweiten Temperatur übergeht.

5. Koaxiale Doppellumenkanülenanordnung (10) nach Anspruch 3, wobei das zweite Profil zur Positionierung in einer Biegung einer Pulmonararterie eines Patienten eingerichtet ist.

6. Koaxiale Doppellumenkanülenanordnung (10) nach Anspruch 1, wobei ein Profil der distalen Spitze (70) eine rohrförmige Struktur definiert, die um eine Achse gekrümmt ist, welche rechtwinklig zu einer Längsachse des ersten länglichen Körpers (28) ist.

7. Koaxiale Doppellumenkanülenanordnung (10) nach Anspruch 6, wobei das Profil der distalen Spitze (70) zum Positionieren in einer Biegung einer Pulmonararterie eines Patienten eingerichtet ist.

8. Koaxiale Doppellumenkanülenanordnung (10) nach Anspruch 1, wobei das distale Ende der distalen Spitze (70) ein Endmerkmal definiert, welches so geformt ist, dass die Prävalenz von Kanten der distalen Spitze (70) reduziert wird, um venöses Einführen der koaxialen Doppellumenkanülenanordnung in den Patienten zu erleichtern und das Risiko von Verletzung der Gefäßwand zu reduzieren.

9. Koaxiale Doppellumenkanülenanordnung (10) nach Anspruch 8, wobei das Endmerkmal ein Profil von mindestens einem von einem Ballon (77), einer Halbkugel, einem Oval und einer Parabel aufweist.

10. Koaxiale Doppellumenkanülenanordnung (10) nach Anspruch 1, wobei die distale Spitze (70) des Weiteren eine Klappe (90) umfasst, die zwischen einer geschlossenen Position, die Fluidfluss aus dem distalen Lumen heraus verhindert, und einer geöffneten Position, die Fluidfluss aus dem distalen Lumen heraus zulässt, bewegbar ist; und wobei in der geschlossenen Position der Klappe (90) Fluidfluss in das distale Lumen hinein Rückdruck erzeugt, der Fluid aus einer oder mehreren der Vielzahl von Infusionsöffnungen (18) in dem ersten länglichen Körper (28) herausdrängt.

11. Koaxiale Doppellumenkanülenanordnung (10) nach Anspruch 10, wobei die Klappe (90) in der geschlossenen Position vorgespannt ist und durch Anwendung einer Kraft auf die distale Seite der Klappe (90) in die geöffnete Position bewegbar ist.

## Revendications

1. Ensemble canule coaxiale à double lumière (10) comprenant :
un premier tube de perfusion (12) ayant un premier corps allongé (28) définissant une première lumière (29) à travers celui-ci, le premier tube de perfusion (12) ayant une extrémité proximale (30), une extrémité distale (32), une première paroi latérale (36) s'étendant de manière circonférentielle entre elles, et une pluralité d'ouvertures de perfusion (18) s'étendant à travers la première paroi latérale (36) au niveau de l'extrémité distale (32) du premier tube de perfusion (12) ;
un second tube de drainage (14) aligné de manière coaxiale avec le premier tube de perfusion (12) et ayant un second corps allongé (40) avec une seconde lumière (46) définie par un espace entre le premier tube de perfusion (12) et le second tube de drainage (14), le second tube de drainage (14) ayant une extrémité proximale (42), une extrémité distale (44), une paroi latérale (48) s'étendant de manière circonférentielle entre elles, et une pluralité d'ouvertures de drainage (20) au niveau de l'extrémité distale (44) du second tube de drainage (14) ; et
un raccord (22) fixé à l'extrémité proximale (30) du premier tube de perfusion (12) et à l'extrémité proximale (42) sur le second tube de drainage (14) ;
dans lequel l'ensemble canule coaxiale à double lumière comprend en outre :
une pointe distale (70) définissant une lumière distale (72) à travers celle-ci,
**caractérisé en ce que**
la pointe distale (70) a une extrémité proximale, une extrémité distale et une paroi latérale (71) s'étendant de manière circonférentielle entre elles, la pointe distale (70) étant reliée à l'extrémité distale (32) du premier corps allongé (28), dans lequel la pointe distale (70) est constituée d'un matériau ayant une dureté inférieure à la dureté du premier corps allongé (28) ; et
dans lequel la première paroi latérale (36) du premier tube de perfusion (12) définit un épaulement (75) de sorte qu'une partie de la première paroi latérale (36) du premier tube de perfusion (12) s'étendant de manière distale depuis l'épaulement (75) présente un diamètre externe inférieur à une partie de la première paroi latérale (36) du premier tube de perfusion (36) s'étendant de manière proximale depuis l'épaulement (75), et la paroi latérale (71) de la pointe distale (70) définit un alésage (76) adapté pour recevoir la partie de la première paroi latérale (36) du premier tube de perfusion (12) s'étendant de manière distale depuis l'épaulement (75), de sorte qu'une partie de la paroi latérale (71) de la pointe distale (70) chevauche la partie de la première paroi latérale (36) du premier tube de perfusion (12) s'étendant de manière distale depuis l'épaulement (75).

2. Ensemble canule coaxiale à double lumière (10) selon la revendication 1, comprenant en outre un panier (80) disposé entre la paroi latérale (71) de la pointe distale (70) et la première paroi latérale (36) du premier tube de perfusion (12), le panier (80) comprenant une pluralité d'éléments individuels (81) agencés de manière à empêcher l'affaissement de la lumière distale.

3. Ensemble canule coaxiale à double lumière (10) selon la revendication 2, dans lequel le panier (80) est constitué d'un matériau à mémoire de forme définissant un premier profil à une première température et un second profil à une seconde température.

4. Ensemble canule coaxiale à double lumière (10) selon la revendication 3, dans lequel le premier profil est un profil tubulaire droit ;
dans lequel la première température est une température à laquelle la pointe distale (70) est reliée à l'extrémité distale (32) du premier corps allongé (28) pendant un procédé de fabrication de l'ensemble canule coaxiale à double lumière (10) ;
dans lequel le second profil est une structure tubulaire s'incurvant autour d'un axe perpendiculaire à un axe longitudinal du premier corps allongé (28) ;
dans lequel la seconde température est une température de corps humain ; et
dans lequel le panier (80) passe du premier profil au second profil à mesure que une température de l'ensemble canule coaxiale à double lumière (10) passe de la première température à la seconde température.

5. Ensemble canule coaxiale à double lumière (10) selon la revendication 3, dans lequel le second profil est adapté pour un positionnement dans une courbure d'une artère pulmonaire d'un patient.

6. Ensemble canule coaxiale à double lumière (10) selon la revendication 1, dans lequel un profil de la pointe distale (70) définit une structure tubulaire s'incurvant autour d'un axe perpendiculaire à un axe longitudinal du premier corps allongé (28).

7. Ensemble canule coaxiale à double lumière (10) selon la revendication 6, dans lequel le profil de la pointe distale (70) est adapté pour un positionnement dans une courbure d'une artère pulmonaire d'un patient.

8. Ensemble canule coaxiale à double lumière (10) selon la revendication 1, dans lequel l'extrémité distale de la pointe distale (70) définit un élément d'extrémité conformé pour réduire la prévalence de bords de la pointe distale (70) afin de faciliter l'insertion veineuse de l'ensemble canule coaxiale à double lumière chez le patient et de réduire le risque de lésion de la paroi vasculaire.

9. Ensemble canule coaxiale à double lumière (10) selon la revendication 8, dans lequel l'élément d'extrémité a un profil d'au moins l'un parmi un ballonnet (77), un hémisphère, un ovale et une parabole.

10. Ensemble canule coaxiale à double lumière (10) selon la revendication 1, dans lequel la pointe distale (70) comprend en outre un volet (90) mobile entre une position fermée empêchant l'écoulement de fluide hors de la lumière distale et une position ouverte permettant l'écoulement de fluide hors de la lumière distale ; et dans lequel, dans la position fermée du volet (90), l'écoulement de fluide dans la lumière distale crée une contre-pression forçant le fluide à sortir d'une ou de plusieurs de la pluralité d'ouvertures de perfusion (18) dans le premier corps allongé (28).

11. Ensemble canule coaxiale à double lumière (10) selon la revendication 10, dans lequel le volet (90) est sollicité dans la position fermée et est mobile vers la position ouverte par l'application d'une force sur un côté distal du volet (90).
